(19) Europäisches Patentamt
European Patent Office
Office européen des brevets

(11) Numéro de publication: **0 013 250**
**B1**

# (12) FASCICULE DE BREVET EUROPEEN

(45) Date de publication du fascicule du brevet: **14.03.84**

(51) Int. Cl.³: **C 07 C 41/16**, C 07 C 43/23

(21) Numéro de dépôt: **79420063.4**

(22) Date de dépôt: **28.11.79**

(54) **Procédé de préparation de monoéthers insaturés de diphénols.**

(30) Priorité: **30.11.78 FR 7834297**

(43) Date de publication de la demande: **09.07.80 Bulletin 80/14**

(45) Mention de la délivrance du brevet: **14.03.84 Bulletin 84/11**

(84) Etats contractants désignés: **AT BE CH DE FR GB IT LU NL SE**

(56) Documents cités:
**FR - A - 1 573 164**
**FR - A - 2 255 279**

**Le dossier contient des informations techniques présentées postérieurement au dépôt de la demande et ne figurant pas dans le présent fascicule.**

(73) Titulaire: **RHONE-POULENC AGROCHIMIE, 14-20, rue Pierre Baizet, F-69009 Lyon (FR)**

(72) Inventeur: **Michelet, Daniel, 24, Chemin de Montribloud, F-69160 Tassin la Demi Lune (FR)**
Inventeur: **Rakoutz, Michel, 6, Boulevard de l'Europe, F-69600 Oullins (FR)**

(74) Mandataire: **Brachotte, Charles et al, RHONE-POULENC AGROCHIMIE BP 9163-09, F-69263 Lyon Cedex 1 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

# Procédé de préparation de monoéthers insaturés de diphénols

La présente invention concerne un procédé de préparation d'éthers insaturés de diphénols.

Il est connu de préparer des éthers insaturés de diphénols par action d'halogénures d'alcényle sur des diphénols en présence d'agents alcalins (brevet français 2 255 279).

Un but de la présente invention est de fournir un procédé amélioré de préparation d'éthers insaturés de diphénols.

Un autre but de l'invention est de préparer des alcényloxyphénols $\beta,\gamma$-insaturés à partir de diphénols et d'halogénures d'alcényle avec des taux de transformation et des rendements améliorés.

Un autre but de l'invention est de préparer préférentiellement des monoéthers de diphénols en évitant au maximum la formation de diéthers (réaction parasite d'O-alkylation) ou d'alcényldiphénols (réaction parasite de C-alkylation).

Un autre but de l'invention est de fournir un procédé de préparation d'alcényloxyphénols permettant l'utilisation de réactifs simples, notamment les dérivés alcalins comme agents basiques.

Il a maintenant été trouvé que ces buts pouvaient être atteints grâce à un nouveau procédé qui fait l'objet de la présente invention.

Ce procédé est un procédé de préparation d'alcényloxyphénol $\beta,\gamma$-insaturé à partir de diphénol et d'halogénure d'alcényle $\beta,\gamma$-insaturé caractérisé en ce que, dans une première zone se trouve un milieu liquide constitué de deux phases liquides, l'une organique, l'autre aqueuse, ces phases étant mélées l'une à l'autre, et le milieu liquide étant obtenu par mélange du diphénol, de l'halogénure d'alcényle $\beta,\gamma$-insaturé, d'un solvant organique, d'un agent basique, d'eau et d'un dérivé d'ammonium ou phosphonium quaternaire, la température de cette première zone étant telle qu'il ne se produit substantiellement aucune réaction chimique de l'halogénure d'alcényle c'est à dire inférieure à 50° C, puis que la phase organique est séparée de la phase aqueuse et est chauffée dans une seconde zone, ce chauffage étant effectué à température suffisante pour que l'halogénure d'alcényle puisse entrer en réaction (c'est à dire comprise entre 50 et 150° C). Ce qui distingue essentiellement la première zone d'avec la seconde est que l'une (la seconde) est à température supérieure à l'autre (la première). Ces deux zones peuvent être distinctes ou confondues au point de vue local mais selon un mode préféré, elles sont distinctes. Dans ce cas, la phase organique est extraite du milieu liquide de la première zone et est ensuite transférée dans la seconde zone.

Selon une variante préférée de l'invention, on procède à un ou plusieurs recyclages du milieu liquide de la seconde zone vers la première zone, ces recyclages pouvant être effectués soit en discontinu soit en continu. On peut ainsi réintroduire dans la première zone tout ou partie du milieu liquide de la seconde zone; plusieurs traitements successifs peuvent être ainsi réalisés alternativement dans la première zone puis dans la seconde zone jusqu'au moment où, ayant assez d'alcényloxyphénol dans la seconde zone on procède à son extraction et/ou séparation par tout moyen connu en soi. Lorsqu'on opère en continu dans des conditions de ce type, on prélève alors en continu une partie de la phase organique de la seconde zone, on introduit cette partie dans la première zone, on extrait en continu une partie de la phase organique de la première zone et on introduit cette partie dans la deuxième zone. Pour économiser de l'énergie, on procède à un échange de calories entre le liquide sortant de la seconde zone et le liquide sortant le la première zone, de manière à conserver la température haute en seconde zone.

Au fur et à mesure du déroulement du procédé de l'invention, soit en continu soit en discontinu, on peut bien entendu rajouter des constituants des milieux liquides et/ou des réactifs aux milieux liquides de la première zone et/ou de la seconde zone. Le diphénol et l'halogénure d'alcényle peuvent ainsi être introduits soit dans la première zone, soit dans la seconde zone; l'agent basique s'ajoute de préférence dans la première zone, et, également de préférence, il n'y a pas de phase aqueuse dans la seconde zone.

Il a été indiqué plus haut que le milieu liquide de la première zone, était obtenu par mélange du diphénol, de l'halogénure d'alcényle $\beta,\gamma$-insaturé, d'un solvant organique, d'un agent basique, d'eau et d'un dérivé d'ammonium ou phosphonium quaternaire; il doit cependant bien être entendu que ces constituants ne sont pas nécessairement introduits dans la première zone directement mais peuvent y être introduits indirectement; par exemple ils peuvent être introduits dans la seconde zone puis le milieu liquide de la seconde zone peut être ultérieurement transféré dans la première zone (l'agent basique toutefois n'est préférentiellement pas introduit dans la seconde zone). Ces divers constituants peuvent être introduits séparément ou concomitamment. Une fois qu'ils ont été introduits il peut se produire des réactions chimiques ou, tout au moins, des échanges équilibrés en sorte que la nature exacte des constituants présents réellement dans les milieux liquides, notamment dans la première zone, n'est pas nécessairement celle des constituants tels qu'ils ont été introduits.

Comme diphénols justiciables du procédé de l'invention, on peut citer la pyrocatéchine, la résorcine et l'hydroquinone. Toutefois la pyrocatéchine est préférée et elle conduit à des orthoalcényloxyphénols.

Comme halogénure d'alcényle $\beta,\gamma$-insaturés on préfère utiliser les chlorures et les bromures de type allylique. Le chlorure de méthallyle se met en œuvre particulièrement bien.

Comme solvant organique on utilise un solvant inerte, c'est-à-dire ne réagissant pas chimiquement dans les conditions du procédé de l'invention, et non miscible à l'eau. Son point d'ébullition est généralement supérieur à 50° C, de préférence supérieur à 70° C. Il est habituellement liquide à 20° C.

Comme solvants utilisables on peut citer: les hydrocarbures aromatiques tels que, par exemple le toluène, les o-, m- et p-xylènes, l'éthylbenzène, le benzène; les hydrocarbures halogénés tels que, par exemple, le dichloro-1,2 éthane, le trichloréthylène, le perchloréthylène, le chlorure de méthallyle, le chlorobenzène, les o-, m- et p-dichlorobenzènes, le trichlorobenzène; les alcools tels que, par exemple les alcools n-amylique, n-hexylique, n-octylique, isoamylique, l'éthyl-2 butanol-1; les éthers tels que, par exemple, l'éther dipropylique, l'éther dibutylique, l'éther dibenzylique, l'éther diisopropylique, le diphényléther, l'anisole, le phénétole, le vératrole; les cétones telles que, par exemple: la méthyl-4 pentanone-2, l'acétophénone, la méthylisobutylcétone; les nitriles tels que, par exemple, le benzonitrile et propionitrile.

L'agent basique est avantageusement un composé minéral soluble dans l'eau. On peut citer les hydroxydes de métaux alcalins (la soude notamment, NaOH) ou alcalino-terreux, les carbonates de métaux alcalins acides ou non, ces composés étant de préférence ceux solubles dans l'eau.

Le dérivé d'ammonium quaternaire est de préférence un sel ou un hydroxyde soluble dans l'eau. Généralement le cation ammonium quaternaire comprend en tout entre 10 et 40 atomes de carbone.

Comme dérivé d'ammonium quaternaire utilisable on peut citer de préférence ceux de formule:

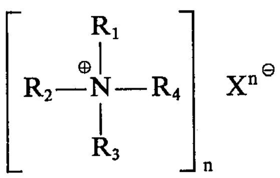

(I)

dans laquelle

- n est un nombre entier positif égal à la valence de l'anion X,
- $R_1$, $R_2$, $R_3$, $R_4$, identiques ou différents, représentent chacun un radical hydrocarboné dont la chaine est éventuellement interrompue par des atomes d'oxygène (chaine oxyalcoylène ou polyoxyalcoylène), ce radical étant éventuellement substitué, notamment par des groupes hydroxyle; de préférence, les radicaux $R_1$, $R_2$, $R_3$, $R_4$ ont au plus 30 atomes de carbone; ils peuvent en particulier être de type alcoyle, alcényle (ou hydroxyalcoyle), phénylalcoyle,
- X est un anion minéral ou organique de valence n. Comme exemple d'anions utilisables on peut citer les ions chlorure, bromure, fluorure, sulfate, sulfate acide, $H_2PO_4^-$, hydroxyle, alcoxysulfonyloxyle (notamment ceux contenant de 1 à 4 atomes de carbone tels que les radicaux méthoxysulfonyloxyle et éthoxysulfonyloxyle), alcanesulfonyloxyle (notamment ceux contenant de 1 à 4 atomes de carbone tels que méthanesulfonyloxyle ou éthanesulfonyloxyle), arylsulfonyloxyle (notamment benzènesulfonyloxyle ou p-toluènesulfonyloxyle) ou alcanoyloxyle contenant de à 4 atomes de carbone (tels que acétyloxyle et propionyloxyle).

Les dérivés d'ammonium quaternaire préférés sont ceux de type alcoyltributylammonium dans lesquels le groupe alcoyle possède de 1 à 4 atomes de carbone.

Comme dérivé d'ammonium quaternaire on peut donc citer plus spécialement les chlorures, hydroxydes ou bisulfates de méthyltributylammonium, éthyltributylammonium, tétrabutylammonium.

Les dérivés de phosphonium utilisables dans l'invention ont pour formule:

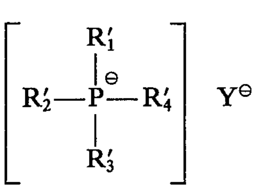

(II)

dans laquelle $R_1'$, $R_2'$, $R_3'$, et $R_4'$ identiques ou différents représentent chacun un radical alcoyle contenant de 2 à 8 atomes de carbone, Y représente un atome de chlore ou de brome.

La température, surtout dans le cas de la mise en œuvre de la pyrocatéchine et du chlorure de méthallyle, est pour la première zone, généralement inférieure à 50° C, de préférence inférieure à 30° C, et pour la seconde zone supérieure à 50° C et inférieure à 150° C, de préférence comprise entre 80 et 120° C. Selon la température il peut bien entendu être nécessaire d'opérer dans un récipient sous pression. Les limites supérieures de température sont principalement liées à la stabilité des réactifs mis en œuvre. Selon un mode de réalisation préféré, la première zone est laissée à température ambiante ou à la température naturelle du mélange sans qu'il soit procédé à aucun chauffage particulier.

La concentration des milieux liquides pour la première zone et la seconde zone (c'est-à-dire la concentration de l'ensemble diphénol + halogénure d'alcényle + agent basique + dérivé d'ammonium ou phosphonium) est généralement comprise entre 5 et 70% en poids, de préférence entre 30 et 60% en poids.

La concentration en dérivé ammonium ou phosphonium par rapport au solvant organique est généralement comprise entre 0,01 et 2 moles par litre de solvant, de préférence entre 0,1 et 1 mole/litre.

Lorsqu'on introduit dans la première zone, soit en continu soit en discontinu (entre 2 recyclages), aussi bien de l'agent basique que tout ou partie du milieu liquide (de préférence refroidi) issu de la 2e zone, alors les quantités respectives de ces deux ajouts sont telles que le rapport molaire

$$\frac{\text{quantité d'agent basique introduit dans la lère zone}}{\text{quantité d'ions halogénures dans le liquide transféré de la 2e zone vers la lère zone}}$$

soit inférieur ou égal à 2, de préférence inférieur ou égal à 1.

Le rapport molaire

$$\frac{\text{quantité d'agent basique introduit}}{\text{quantité de diphénol introduit}}$$

est généralement compris entre 0,1 et 1, de préférence compris entre 0,4 et 0,7. Dans une opération continue les quantités introduites figurant dans ce rapport sont des quantités instantanées, autrement dit des débits molaires. Dans une opération en discontinu on prend en considération la totalité de la quantité introduite depuis le début de l'opération.

La première zone est avantageusement située dans un mélangeur-décanteur ou dans une colonne de lavage liquide/liquide fonctionnant à contre-courant.

Les alcényloxyphénols sont utiles comme intermédiaires pour la fabrication d'aromes et parfums. Les orthoalcényloxyphénols sont utiles comme intermédiaires pour la préparation de dérivés de benzofuranes à propriétés insecticides.

Le procédé de l'invention est particulièrement intéressant en raison des bons résultats obtenus.

Les exemples suivants donnés à titre non limitatif illustrent l'invention et montrent comment elle peut être mise en pratique.

## Exemple 1

Dans un ballon A équipé d'un agitateur magnétique, on charge à 18° C:

- 70 $cm^3$ d'anisole dégazée à l'aide d'argon,
- 16,5 g de pyrocatéchine,
- 20 $cm^3$ de chlorure de méthallyle,
- 10 g de chlorure de tétrabutylammonium,
- une solution aqueuse obtenue à l'aide de 1,49 g de NaOH et 10 $cm^3$ d'eau.

La température s'élève de 18 à 27° C lors de l'addition des réactifs.

L'agitation est poursuivie pendant 20 minutes; on laisse décanter, puis la phase organique est transférée dans un ballon B identique à A ayant une atmosphère d'argon. On chauffe le ballon B 30 minutes à 100° C, refroidit à température ambiante et réintroduit son contenue dans le ballon A.

On ajoute dans le ballon A une solution de 1,26 g de soude dans 5 $cm^3$ d'eau et le même cycle d'opérations est recommencé une fois; on ajoute dans le ballon A une solution de 1,29 g de soude dans 5 $cm^3$ d'eau et le même cycle d'opérations est recommencé deux fois sans recharger de la soude entre ces deux derniers cycles d'opérations.

On réunit alors le contenu des ballons A et B, on acidifie jusqu'à pH = 4 (pour la phase aqueuse) avec une solution aqueuse d'acide sulfurique N.

On extrait à l'éther en effectuant des lavages à l'eau.

On obtient finalement

- 9,15 g de pyrocatéchine restante,
- 10 g d'orthométhallyloxyphénol (rendement de 91,3% par rapport à la pyrocatéchine transformée),
- 0,08 g d'orthométhallylpyrocatéchine (rendement de 0,7% par rapport à la pyrocatéchine transformée).

Exemple 2

Dans un ballon A équipé d'un agitateur magnétique, on charge à 18° C:

- 350 $cm^3$ d'anisole,
- 100 $cm^3$ de chlorure de méthallyle,
- 50 g de chlorure de tétrabutylammonium,
- 45 g de pyrocatéchine,
- 50 $cm^3$ d'une solution aqueuse contenant 7,2 g de soude (NaOH).

On agite 20 minutes dans le ballon A puis on décante et la phase organique est transférée dans le ballon B où elle est chauffée 30 minutes à 100° C; on refroidit à température ambiante et réintroduit le contenu du ballon B dans le ballon A.

Dans ce ballon A, on ajoute: 16,5 g de pyrocatéchine et une solution de 6 g de soude dans 25 $cm^3$ d'eau et on recommence une fois le même cycle d'opérations. On ajoute dans le ballon A 16,5 g de pyrocatéchine et une solution de 6 g de soude dans 25 $cm^3$ d'eau et on recommence deux fois le même cycle d'opérations sans rien recharger entre ces deux derniers cycles d'opérations.

On obtient finalement les résultats suivants:

- 31,56 g de pyrocatéchine restante (taux de transformation 59,5%),
- 61,5 g d'orthométhallyloxyphénol (rendement de 89% par rapport à la pyrocatéchine transformée),
- 0,26 g d'orthométhallylpyrocatéchine (rendement de 0,4% par rapport à la pyrocatéchine transformée).

Exemple 3

Dans un ballon A équipé d'un agitateur magnétique, on charge à 18° C:

- 70 $cm^3$ d'anisole,
- 14,5 g de pyrocatéchine,
- 10 g de chlorure de tétrabutylammonium,
- 10 $cm^3$ d'une solution aqueuse contenant 1,48 g de soude.

Après 20 minutes d'agitation, on décante et la phase organique est transférée dans le ballon B; dans ce dernier ballon B on ajoute 10 $cm^3$ de chlorure de méthallyle et on chauffe 30 minutes à 100° C. Après refroidissement on réintroduit le contenu du ballon B dans le ballon A et on recommence successivement 3 cycles d'opération similaires.

On indique ci-après, pour chaque cycle, la quantité de soude introduite dans le ballon A avant agitation et la quantité de chlorure de méthallyle introduite dans le ballon B avant chauffage étant entendu que chaque cycle reprend les opérations qui viennent d'être décrites.

| N° du cycle | soude en g | chlorure de méthallyle en $cm^3$ |
|---|---|---|
| 1 | 1,48 | 10 |
| 2 | 1,34 | 3,5 |
| 3 | 1,28 | 3,5 |
| 4 | 0 | 3,5 |

On obtient finalement

- 5,73 g de pyrocatéchine restante (taux de transformation: 60,5%),
- 11,9 g d'orthométhallyloxyphénol (rendement 91% par rapport à la pyrocatéchine transformée),
- 0,08 g d'orthométhallylpyrocatéchine (rendement 0,6% par rapport à la pyrocatéchine transformée).

Exemple 4

Dans un ballon A équipe d'un agitateur magnétique, on charge à 18° C:

- 70 $cm^3$ d'anisole,
- 20 $cm^3$ de chlorure de méthallyle,
- 10 g de chlorure de tétrabutylammonium,
- 9 g de pyrocatéchine,
- 10 $cm^3$ d'une solution aqueuse contenant 1,5 g de soude.

Après agitation 30 minutes à 25° C et décantation, la phase organique est transférée dans un ballon B chauffé 1 h 30 min à 90° C.

Après refroidissement, on ajoute et dissout 3,27 g de pyrocatéchine et on réintroduit l'ensemble dans le ballon A où on ajoute, en outre, 1,19 g de soude dissoute dans 5 $cm^3$ d'eau. On recommence le cycle d'opérations déjà indiqué deux fois, étant entendu qu'entre les deux derniers cycles d'opérations on ajoute 3,38 g de pyrocatéchine dans le ballon B et 1,26 g de soude (dans 5 $cm^3$ d'eau) dans le ballon A.

On obtient finalement:

- pyrocatéchine restante:
  6,67 g
- orthométhallyloxyphénol:
  12,11 g (rendement 90,5% par rapport à la pyrocatéchine transformée).

**Revendications**

1. Procédé de préparation d'alcényloxyphénol $\beta,\gamma$-insaturé à partir de diphénol et d'halogénure d'alcényle $\beta,\gamma$-insaturé caractérisé en ce que, dans une première zone se trouve un milieu liquide constitué de deux phases liquides, l'une organique, l'autre aqueuse, ces phases étant mêlées l'une à l'autre, et le milieu liquide étant obtenu par mélange du diphénol, de l'halogénure d'alcényle $\beta,\gamma$-insaturé, d'un solvant organique, d'un agent basique, d'eau et d'un dérivé d'ammonium ou phosphonium quaternaire, la température de cette première zone étant inférieure à 50° C, et étant telle qu'il ne se produit substantiellement aucune réaction chimique de l'halogénure d'alcényle, puis que la phase organique est séparée de la phase aqueuse et est chauffée dans une seconde zone, ce chauffage étant effectué à température comprise entre 50 et 150° C, cette température étant suffisante pour que l'halogénure d'alcényle puisse entrer en réaction.

2. Procédé selon la revendication 1 caractérisé en ce que tout ou partie du milieu liquide de la seconde zone est refroidi et introduit dans la première zone.

3. Procédé selon la revendication 2 caractérisé en ce que l'on ajoute à nouveau dans la première zone de l'agent basique et éventuellement du diphénol et/ou de l'halogénure d'alcényle $\beta,\gamma$-insaturé, ces deux réactifs étant ajoutés soit dans la première zone, soit dans la seconde.

4. Procédé selon l'une des revendications 1 à 3 caractérisé en ce que l'alcényloxyphénol $\beta,\gamma$-insaturé formé est extrait du milieu liquide de la seconde zone.

5. Procédé selon l'une des revendications 1 à 4 caractérisé en ce que l'on prélève en continu une partie de la phase organique de la seconde zone, et que l'on introduit cette partie dans la première zone, et on extrait en continu une partie de la phase organique de la première zone, et qu'on introduit cette partie dans la deuxième zone.

6. Procédé selon la revendication 5 caractérisé en ce que l'agent basique est introduit en continu dans la première zone.

7. Procédé selon l'une des revendications 1 à 6 caractérisé en ce que le diphénol est la pyrocatéchine.

8. Procédé selon l'une des revendications 1 à 7 caractérisé en ce que l'halogénure d'alcényle est le chlorure de méthallyle.

9. Procédé selon les revendications 7 et 8 caractérisé en ce que la température de la première zone est inférieure à 30° C et que la température de la seconde zone est comprise entre 80 et 120° C.

10. Procédé selon l'une des revendications 1 à 9 caractérisé en ce que le solvant organique est un solvant non miscible à l'eau, de point d'ébullition supérieur à 50° C, liquide à 20° C et est un hydrocarbure aromatique ou un hydrocarbure halogéné ou un alcool ou un éther ou une cétone ou un nitrile.

11. Procédé selon l'une des revendications 1 à 10 caractérisé en ce que l'agent basique est un hydroxyde ou carbonate alcalin.

12. Procédé selon l'une des revendications 1 à 11 caractérisé en ce que le dérivé d'ammonium quaternaire est un hydroxyde ou un sel, soluble dans l'eau et dont la partie cationique comprend entre 10 et 40 atomes de carbone.

13. Procédé selon l'une des revendications 1 à 12 caractérisé en ce que la concentration des milieux liquides pour la 1 ère et la 2ème zone est comprise entre 5 et 70% et que la concentration en dérivé d'ammonium ou phosphonium par rapport au solvant organique est comprise entre 0,01 et 2 moles/litre.

14. Procédé selon la revendication 13 caractérisé en ce que la concentration des milieux liquides est comprise entre 30 et 60% ou que la concentration en dérivé ammonium ou phosphonium est comprise entre 0,1 et 1 mole/litre.

15. Procédé selon l'une des revendications 1 à 14 caractérisé en ce que le rapport molaire

$$\frac{\text{quantité d'agent basique introduite}}{\text{quantité de diphénol introduite}}$$

est compris entre 0,1 et 1.

16. Procédé selon la revendication 15 caractérisé en ce que ce rapport est compris entre 0,4 et 0,7.

**Patentansprüche**

1. Verfahren zur Herstellung von $\beta,\gamma$-ungesättigten Alkenyloxyphenolen, ausgehend von zweiwertigem Phenol und $\beta,\gamma$-ungesättigtem Alkenylhalogenid, dadurch gekennzeichnet, daß in einer ersten Zone ein flüssiges Medium vorhanden ist, das aus zwei flüssigen Phasen besteht, von denen die eine organisch und die andere wäßrig ist, wobei diese Phasen miteinander vermischt sind und das flüssige Medium durch Vermischen von zweiwertigem Phenol, $\beta,\gamma$-ungesättigtem Alkenylhalogenid, organischem Lösungsmittel, basischem Mittel, Wasser und einer quaternären Ammonium- oder Phosphoniumverbindung erhalten worden ist und die Temperatur dieser ersten Zone unter 50°C ist und bei der Temperatur dieser ersten Zone praktisch keinerlei chemische Reaktion des Alkenylhalogenids stattfindet, daß die organische Phase dann von der wäßrigen Phase abgetrennt und in einer zweiten Zone erhitzt wird, auf eine zwischen 50°C und 150°C ausreichende Temperatur, damit das Alkenylhalogenid reagieren kann.

2. Anspruch nach Punkt 1, gekennzeichnet dadurch, daß die Gesamtmenge oder ein Teil des flüssigen Mediums der zweiten Zone abgekühlt wird und in die erste Zone eingebracht wird.

3. Anspruch nach Punkt 2, gekennzeichnet dadurch, daß man erneut in die erste Zone das basische Mittel und gegebenenfalls zweiwertiges Phenol und/oder $\beta,\gamma$-ungesättigtes Alkenylhalogenid einbringt, wobei die beiden letzteren Reaktionspartner entweder der ersten Zone oder der zweiten Zone zugefügt werden.

4. Anspruch nach einem der Punkte 1 bis 3, gekennzeichnet dadurch, daß das entstandene $\beta,\gamma$-ungesättigte Alkenyloxyphenol aus dem flüssigen Medium der zweiten Zone extrahiert wird.

5. Anspruch nach einem der Punkte 1 bis 4, gekennzeichnet dadurch, daß man kontinuierlich einen Teil der organischen Phase der zweiten Zone entnimmt, diesen Teil in die erste Zone einleitet und daß man kontinuierlich einen Teil der organischen Phase der ersten Zone abzieht und diesen Teil in die zweite Zone einführt.

6. Anspruch nach Punkt 5, gekennzeichnet dadurch, daß das basische Mittel kontinuierlich in die erste Zone eingebracht wird.

7. Anspruch nach einem der Punkte 1 bis 6, gekennzeichnet dadurch, daß das zweiwertige Phenol Brenzcatechin ist.

8. Anspruch nach einem der Punkte 1 bis 7, gekennzeichnet dadurch, daß das Alkenylhalogenid Metallylchlorid ist.

9. Anspruch nach den Punkten 7 und 8, gekennzeichnet dadurch, daß die Temperatur der ersten Zone unterhalb 30°C und die Temperatur der zweiten Zone zwischen 80 und 120°C liegt.

10. Anspruch nach einem der Punkte 1 bis 9, gekennzeichnet dadurch, daß das organische Lösungsmittel ein mit Wasser nicht mischbares Lösungsmittel mit Siedepunkt über 50°C ist, das bei 20°C flüssig und ein aromatischer Kohlenwasserstoff oder ein Halogenkohlenwasserstoff oder ein Alkohol oder ein Äther oder ein Keton oder ein Nitril ist.

11. Anspruch nach einem der Punkte 1 bis 10, gekennzeichnet dadurch, daß das basische Mittel ein Alkalihydroxyd oder Alkalicarbonat ist.

12. Anspruch nach einem der Punkte 1 bis 11, gekennzeichnet dadurch, daß die quaternäre Ammoniumverbindung ein wasserlösliches Hydroxyd oder Salz ist, dessen kationischer Teil 10 bis 40 Kohlenstoffatome enthält.

13. Anspruch nach einem der Punkte 1 bis 12, gekennzeichnet dadurch, daß die Konzentration der flüssigen Medien in der ersten und in der zweiten Zone 5 bis 70% ausmacht und daß die Konzentration an Ammonium- oder Phosphoniumverbindung, bezogen auf organisches Lösungsmittel, 0,01 bis 2 Mol/l ausmacht.

14. Anspruch nach Punkt 13, gekennzeichnet dadurch, daß die Konzentration der flüssigen Medien 30 bis 60% ausmacht und daß die Konzentration an Ammonium- oder Phosphoniumverbindung 0,1 bis 1 Mol/l beträgt.

15. Anspruch nach einem der Punkte 1 bis 14, gekennzeichnet dadurch, daß das Molverhältnis von Menge an zugeführtem basischem Mittel zu Menge an zugeführtem zweiwertigem Phenol 0,1 bis 1 beträgt.

16. Anspruch nach Punkt 15, gekennzeichnet dadurch, daß dieses Verhältnis 0,4 bis 0,7 ausmacht.

## Claims

1. A process for the preparation of a $\beta,\gamma$-unsaturated alkenyloxyphenol from a dihydric phenol and a $\beta,\gamma$-unsaturated alkenyl halide wherein, in a first zone, there is a liquid medium consisting of two liquid phases of which one is organic and the other is aqueous, these phases being mixed with one another, and the liquid medium being obtained by mixing the dihydric phenol, the $\beta,\gamma$-unsaturated alkenyl halide, an organic solvent, a basic agent, water and a quaternary ammonium or phosphonium derivative, the temperature of this first zone being below 50° C and such that substantially no chemical reaction of the alkenyl halide takes place, and wherein the organic phase is then separated from the aqueous phase and is heated in a second zone, this heating being effected between 50 and 150° C at a temperature which is sufficient for the alkenyl halide to react with the dihydric phenol.

2. A process according to claim 1, wherein all or part of the liquid medium in the second zone is cooled and introduced into the first zone.

3. A process according to claim 2, wherein a further amount of the dihydric phenol and/or the $\beta,\gamma$-unsaturated alkenyl halide is added to the first zone or to the second zone.

4. A process according to any one of claims 1 to 3, wherein the $\beta,\gamma$-unsaturated alkenyloxyphenol formed is extracted from the liquid medium in the second zone.

5. A process according to any one of claims 1 to 4, wherein part of the organic phase is removed continuously from the second zone, this part is introduced into the first zone, part of the organic phase is extracted continuously from the first zone and this part is introduced into the second zone.

6. A process according to claim 5, wherein the basic agent is introduced continuously into the first zone.

7. A process according to any one of claims 1 to 6, wherein the dihydric phenol is pyrocatechol.

8. A process according to any one of claims 1 to 7, wherein the alkenyl halide is methallyl chloride.

9. A process according to claims 7 and 8, wherein the temperature of the first zone is below 30° C and the temperature of the second zone is between 80 and 120° C.

10. A process according to anyone of claims 1 to 9, wherein the inert organic solvent immiscible with water has a boiling point above 50°C, is liquid at 20°C and is an aromatic hydrocarbon, a halogenohydrocarbon, an alcohol, an ether, a ketone or a nitrile.

11. A process according to any one of claims 1 to 10, wherein the basic agent is an alkali metal hydroxide or carbonate.

12. A process according to any one of claims 1 to 11, wherein the quaternary ammonium derivative is a water-soluble hydroxide or salt, the cationic part of which contains between 10 and 40 carbon atoms.

13. A process according to any one of claims 1 to 12, wherein the concentration of the liquid media for the first and the second zones is between 5 and 70% and the concentration of ammonium or phosphonium derivative, relative to the organic solvent, is between 0.01 and 2 mols/litre.

14. A process according to claim 13, wherein the concentration of the liquid media is between 30 and 60% or the concentration of ammonium or phosphonium derivative is between 0.1 and 1 mol/litre.

15. A process according to any one of claims 1 to 14, wherein the molar ratio

$$\frac{\text{amount of basic agent introduced}}{\text{amount of dihydric phenol introduced}}$$

is between 0.1 and 1.

16. A process according to claim 15 wherein the said ratio is between 0.4 and 0.7.